(19) Europäisches Patentamt
European Patent Office
Office européen des brevets

(11) **EP 3 293 516 B1**

(12) **EUROPEAN PATENT SPECIFICATION**

(45) Date of publication and mention
of the grant of the patent:
**15.05.2019 Bulletin 2019/20**

(51) Int Cl.:
**G01N 27/406** (2006.01)    **G01N 27/417** (2006.01)
**G01N 27/419** (2006.01)    **G01N 33/00** (2006.01)

(21) Application number: **17189261.5**

(22) Date of filing: **04.09.2017**

(54) **CONCENTRATION COMPUTATION APPARATUS**

KONZENTRATIONSBERECHNUNGSVORRICHTUNG

APPAREIL DE CALCUL DE CONCENTRATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **08.09.2016 JP 2016175527**

(43) Date of publication of application:
**14.03.2018 Bulletin 2018/11**

(73) Proprietor: **NGK Spark Plug Co., Ltd.
Nagoya-shi,
Aichi 4678525 (JP)**

(72) Inventors:
• **TOMIMATSU, Chihiro**
**Nagoya-shi, Aichi 4678525 (JP)**
• **TODA, Satoru**
**Nagoya-shi, Aichi 4678525 (JP)**
• **NAKANO, Yoshihiro**
**Nagoya-shi, Aichi 4678525 (JP)**

(74) Representative: **J A Kemp
14 South Square
Gray's Inn
London WC1R 5JJ (GB)**

(56) References cited:
**DE-A1-102016 202 218    US-A1- 2015 013 431
US-A1- 2015 212 035**

**Description**

The present disclosure relates to a multi-gas detection apparatus, in particular, a concentration computation apparatus which computes the concentrations of gas components through use of a multi-gas sensor.

As disclosed in Japanese Patent Application Laid-Open (*kokai*) No. 2015-34814, there has been known an apparatus for computing the concentrations of ammonia, nitrogen dioxide, and nitrogen oxide contained in a measurement gas (or target gas) through use of a multi-gas sensor which includes an NOx detection section and an ammonia detection section for respectively detecting the concentrations of nitrogen oxide and ammonia contained in the measurement gas.

The apparatus disclosed in Japanese Patent Application Laid-Open (*kokai*) No. 2015-34814 has a problem that, in an environment in which the concentrations of the gas components contained in the measurement gas change, the accuracy of the computed gas concentrations decreases.

It is an object of the present disclosure to improve the computation accuracy of the gas concentrations.

The invention provides a concentration computation apparatus according to claim 1.

The speed of response of the first pumping current is the time necessary for the first pumping current to change its value in response to a change in the oxygen concentration in the target gas. The speed of response of the concentration signals is the time necessary for the concentration signals to change their values in response to changes in the ammonia concentration and the nitrogen dioxide concentration in the target gas. Also, the speed of response of the second pumping current is the time necessary for the second pumping current to change its value in response to a change in the nitrogen oxide concentration in the target gas.

In the multi-gas sensor in which the first detection section and the second detection section are united, the first detection section and the second detection section are disposed at approximately the same position in relation to the measurement chamber through which the target gas flows. In such a configuration, there is a tendency that, of the response speeds of the first pumping current, the second pumping current, and the concentration signals, the response speed of the first pumping current is the highest, the response speed of the concentration signals is the second highest, and the response speed of the second pumping current is the lowest.

As a result of the configuration of the first delaying section, when computing the ammonia concentration and the nitrogen dioxide concentration by using the value of the first pumping current and the value of the concentration signals, the concentration computation apparatus can suppress the influence of the difference between the response speed of the first pumping current and the response speed of the concentration signals, to thereby improve the computation accuracy of the ammonia concentration and the nitrogen dioxide concentration.

Further, as a result of the configuration of the second delaying section, when computing the nitrogen oxide concentration by using the ammonia concentration and the nitrogen dioxide concentration, the concentration computation apparatus can suppress the influence of the difference between the response speed of the concentration signals and the response speed of the second pumping current, to thereby improve the computation accuracy of the nitrogen oxide concentration.

In the concentration computation apparatus according to one aspect of the present disclosure, at least one of the digital filter processing performed by the first delaying section and the digital filter processing performed by the second delaying section may be weighted averaging. In this case, since weighted averaging is simple digital filter processing, the processing load of the concentration computation apparatus can be reduced.

These and other features and advantages of the present invention will become more readily appreciated when considered in connection with the following detailed description and appended drawings, wherein like designations denote like elements in the various views, and wherein:FIG. 1 is a cross-sectional view showing the internal structure of a multi-gas sensor 2.FIG. 2 is an illustration showing the schematic structures of a sensor element unit 5 and a control section 3.FIG. 3 is a cross-sectional view showing the structure of a first ammonia detection section 102 and second ammonia detection section 103.FIG. 4 is a functional block diagram showing the outline of processing performed by a microcomputer 190 of a first embodiment.FIG. 5 is a functional block diagram showing the outline of processing performed by a microcomputer 190 of a second embodiment.

(First Embodiment)

A first embodiment of the present disclosure will be described with reference to the drawings.

A multi-gas detection apparatus 100 of the present embodiment is used for a urea SCR system which is mounted on a vehicle and which cleans nitrogen oxide contained in exhaust gas discharged from a diesel engine. More specifically, the multi-gas detection apparatus detects the concentrations of ammonia, nitrogen dioxide, and nitrogen oxide contained in the exhaust gas. The vehicle on which the multi-gas detection apparatus is mounted is referred to as the "present vehicle." Nitrogen dioxide and nitrogen oxide are denoted as $NO_2$ and NOx, respectively. Also, SCR is an abbreviation for selective catalytic reduction.

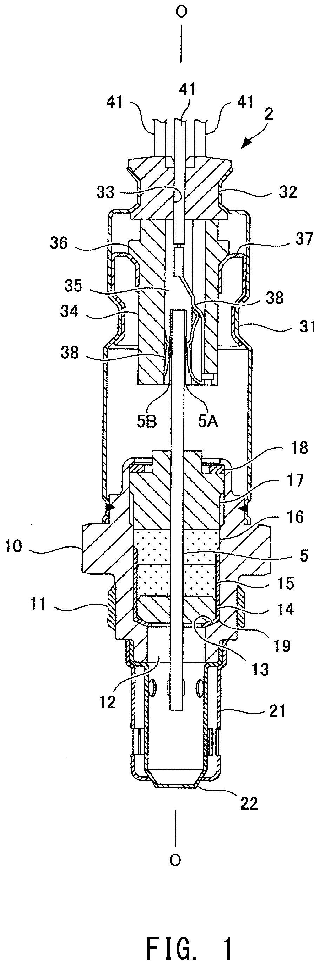

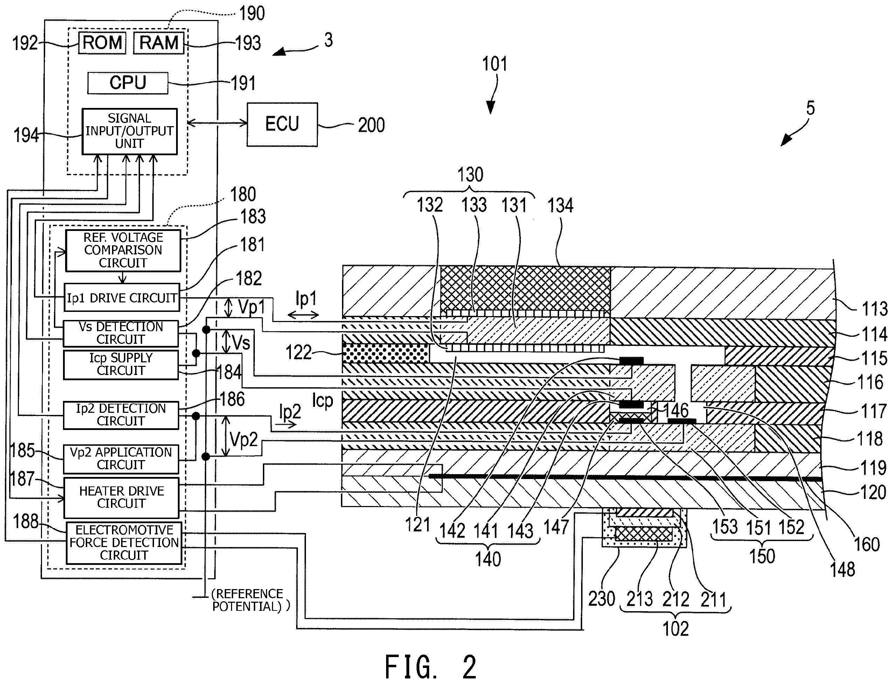

The multi-gas detection apparatus 100 includes a multi-gas sensor 2 shown in FIG. 1 and a control unit 3 shown in FIG. 2.

As shown in FIG. 1, the multi-gas sensor 2 includes a sensor element unit 5, a metallic shell 10, a separator 34, and connection terminals 38. In the following description, the side of the multi-gas sensor 2 on which the sensor element unit 5 is disposed (i.e., the lower side in FIG. 1) is referred to as a forward end side, and the side on which the connection terminals 38 are disposed (i.e., the upper side in FIG. 1) is referred to as a rear end side.

The sensor element unit 5 has a plate shape extending in the direction of an axial line O. Electrode terminal portions 5A and 5B are disposed at the rear end of the sensor element unit 5. In FIG. 1, only the electrode terminal portions 5A and 5B are shown as electrode terminal portions formed in the sensor element unit 5 for the purpose of simplifying the drawing. However, in actuality, a plurality of electrode terminal portions are formed according to the number of, for example, electrodes included in an NOx detection section 101, a first ammonia detection section 102, and a second ammonia detection section 103 described later.

The metallic shell 10 is a tubular member, and a threaded portion 11 used to fix the multi-gas sensor 2 to an exhaust pipe of the diesel engine is formed on the external surface of the metallic shell 10. The metallic shell 10 has a through hole 12 extending in the direction of the axial line O and a ledge 13 protruding inward in the radial direction of the through hole 12. The ledge 13 is formed as an inward tapered surface extending from the radially outer side of the through hole 12 toward its center and inclined toward the forward end side.

The metallic shell 10 holds the sensor element unit 5 such that a forward end portion of the sensor element unit 5 protrudes forward from the through hole 12 and a rear end portion of the sensor element unit 5 protrudes rearward from the through hole 12.

A ceramic holder 14 that is a tubular member surrounding the radial circumference of the sensor element unit 5, talc rings 15 and 16 that are layers of charged talc powder, and a ceramic sleeve 17 are stacked in this order inside the through hole 12 of the metallic shell 10 from the forward end side toward the rear end side.

A crimp packing 18 is disposed between the ceramic sleeve 17 and a rear end portion of the metallic shell 10. A metallic holder 19 is disposed between the ceramic holder 14 and the ledge 13 of the metallic shell 10. The talc ring 15 and the ceramic holder 14 are contained inside the metallic holder 19, and as a result of compressive charging of the material of the talc ring 15, the metallic holder 19 and the talc ring 15 are hermetically integrated together. A rear end portion of the metallic shell 10 is crimped so as to press the ceramic sleeve 17 toward the forward end side through the crimp packing 18. Since the material of the talc ring 16 is compressively charged into the metallic shell 10, the hermetic seal between the inner circumferential surface of the metallic shell 10 and the outer circumferential surface of the sensor element unit 5 is ensured.

An outer protector 21 with gas introduction holes and an inner protector 22 with gas introduction holes are disposed at a forward end portion of the metallic shell 10. The outer protector 21 and the inner protector 22 are each a tubular member formed from a metallic material such as stainless steel and having a closed forward end. The inner protector 22 covers a forward end portion of the sensor element unit 5 and is welded to the metallic shell 10, and the outer protector 21 covers the inner protector 22 and is welded to the metallic shell 10.

A forward end portion of an outer tube 31 formed into a tubular shape is welded to a rear outer circumference of the metallic shell 10. A grommet 32 is disposed in a rear end opening of the outer tube 31 so as to close the opening.

Lead wire insertion holes 33 into which lead wires 41 are inserted are formed in the grommet 32. The lead wires 41 are electrically connected to the electrode terminal portions 5A and 5B of the sensor element unit 5.

The separator 34 is a tubular member disposed rearward of the sensor element unit 5. A space formed inside the separator 34 is an insertion hole 35 passing through the separator 34 in the direction of the axial line O. A flange portion 36 protruding radially outward is formed on the outer surface of the separator 34.

A rear end portion of the sensor element unit 5 is inserted into the insertion hole 35 of the separator 34, and the electrode terminal portions 5A and 5B are disposed inside the separator 34.

A tubular metallic holding member 37 is disposed between the separator 34 and the outer tube 31. The holding member 37 is in contact with the flange portion 36 of the separator 34 and also with the inner surface of the outer tube 31 and thereby holds the separator 34 such that the separator 34 is fixed to the outer tube 31.

The connection terminals 38 are members disposed inside the insertion hole 35 of the separator 34 and are electrically conductive members that electrically connect the electrode terminal portions 5A and 5B of the sensor element unit 5 to their respective lead wires 41. In FIG. 1, only two connection terminals 38 are shown for the purpose of simplifying the drawing.

As shown in FIG. 2, the control section 3 of the multi-gas detection apparatus 100 is electrically connected to an electronic controller 200 mounted on the present vehicle. The electronic controller 200 receives data representing the concentration of $NO_2$, the concentration of NOx, and the concentration of ammonia (hereinafter referred to as the concentration of $NH_3$) in exhaust gas that are computed by the control section 3. Then the electronic controller 200 performs processing for controlling the operating conditions of the diesel engine on the basis of the data received and also performs cleaning processing for NOx accumulated in the catalyst.

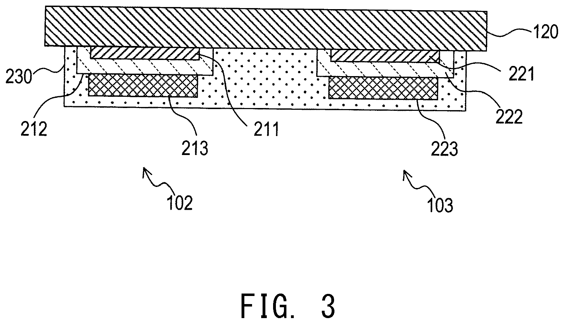

The sensor element unit 5 includes the NOx detection section 101, the first ammonia detection section 102,

and the second ammonia detection section 103. The second ammonia detection section 103 is not shown in FIG. 2 but is shown in FIG. 3. The first ammonia detection section 102 and the second ammonia detection section 103 are disposed in parallel at approximately the same position as a reference electrode 143 in the longitudinal direction of the NOx detection section 101 (i.e., the horizontal direction in FIG. 2) in such a manner that the first ammonia detection section 102 and the second ammonia detection section 103 are located at different positions in the width direction of the NOx detection section 101 (i.e., the direction perpendicular to the sheet on which FIG. 2 is depicted). Therefore, in FIG. 2, of the first ammonia detection section 102 and the second ammonia detection section 103, only the first ammonia detection section 102 is shown.

The NOx detection section 101 is formed by sequentially stacking an insulating layer 113, a ceramic layer 114, an insulating layer 115, a ceramic layer 116, an insulating layer 117, a ceramic layer 118, an insulating layer 119, and an insulating layer 120. The insulating layers 113, 115, 117, 119, and 120 and the ceramic layers 114, 116, and 118 are formed mainly of alumina.

The NOx detection section 101 includes a first measurement chamber 121 formed between the ceramic layer 114 and the ceramic layer 116. In the NOx detection section 101, the exhaust gas is introduced from the outside into the interior of the first measurement chamber 121 through a diffusion resistor 122 that is disposed between the ceramic layer 114 and the ceramic layer 116 so as to be adjacent to the first measurement chamber 121. The diffusion resistor 122 is formed of a porous material such as alumina.

The NOx detection section 101 further includes a first pumping cell 130. The first pumping cell 130 includes a solid electrolyte layer 131 and pumping electrodes 132 and 133.

The solid electrolyte layer 131 is formed mainly of zirconia having oxygen ion conductivity. A part of the ceramic layer 114 is removed from a region exposed to the first measurement chamber 121. Instead of the ceramic layer 114, the solid electrolyte layer 131 is charged (embedded) in the resulting space.

The pumping electrodes 132 and 133 are formed mainly of platinum. The pumping electrode 132 is disposed on a surface of the solid electrolyte layer 131, which surface is exposed to the first measurement chamber 121. The pumping electrode 133 is disposed on the solid electrolyte layer 131 on the side opposite the pumping electrode 132 with the solid electrolyte layer 131 sandwiched between the pumping electrodes 132 and 133. The insulating layer 113 is removed from a region in which the pumping electrode 133 is disposed and from a region around the pumping electrode 133, and the resulting space is filled with a porous material 134 instead of the insulating layer 113. The porous material 134 allows gas (e.g., oxygen) migration between the pumping electrode 133 and the outside.

The NOx detection section 101 further includes an oxygen concentration detection cell 140. The oxygen concentration detection cell 140 includes a solid electrolyte layer 141, a detection electrode 142, and the reference electrode 143.

The solid electrolyte layer 141 is formed mainly of zirconia having oxygen ion conductivity. A part of the ceramic layer 116 is removed from a region on the rear end side (i.e., the right side of FIG. 2) of the solid electrolyte layer 131. Instead of the ceramic layer 116, the solid electrolyte layer 141 is charged (embedded) in the resulting space.

The detection electrode 142 and the reference electrode 143 are formed mainly of platinum. The detection electrode 142 is disposed on a surface of the solid electrolyte layer 141, which surface is exposed to the first measurement chamber 121. The reference electrode 143 is disposed on the solid electrolyte layer 141 on the side opposite the detection electrode 142 with the solid electrolyte layer 141 sandwiched between the detection electrode 142 and the reference electrode 143.

The NOx detection section 101 includes a reference oxygen chamber 146. The reference oxygen chamber 146 is a through hole formed by removing the insulating layer 117 from a region in which the reference electrode 143 is disposed and from a region around the reference electrode 143.

The NOx detection section 101 includes a second measurement chamber 148 disposed downstream of the first measurement chamber 121. The second measurement chamber 148 is formed rearward of the detection electrode 142 and the reference electrode 143 so as to pass through the solid electrolyte layer 141 and the insulating layer 117. In the NOx detection section 101, the exhaust gas discharged from the first measurement chamber 121 is introduced into the second measurement chamber 148.

The NOx detection section 101 includes a second pumping cell 150. The second pumping cell 150 includes a solid electrolyte layer 151 and pumping electrodes 152 and 153.

The solid electrolyte layer 151 is formed mainly of zirconia having oxygen ion conductivity. The ceramic layer 118 is removed from a region exposed to the reference oxygen chamber 146 and the second measurement chamber 148 and a region around this exposed region. Instead of the ceramic layer 118, the solid electrolyte layer 151 is charged (embedded) in the resulting space.

The pumping electrodes 152 and 153 are formed mainly of platinum. The pumping electrode 152 is disposed on a surface of the solid electrolyte layer 151, which surface is exposed to the second measurement chamber 148. The pumping electrode 153 is disposed on the solid electrolyte layer 151 such that the pumping electrode 153 faces the reference electrode 143 with the reference oxygen chamber 146 therebetween. A porous material 147 is disposed inside the reference oxygen chamber 146 so as to cover the pumping electrode 153.

The NOx detection section 101 includes a heater 160. The heater 160 is a heat-generating resistor that is formed mainly of platinum and generates heat when energized and is disposed between the insulating layers 119 and 120.

The first ammonia detection section 102 is formed on the outer surface of the NOx detection section 101, more specifically on the insulating layer 120. The first ammonia detection section 102 is disposed at approximately the same position, with respect to the direction of the axial line O (i.e., the horizontal direction in FIG. 2), as the reference electrode 143 in the NOx detection section 101.

The first ammonia detection section 102 includes a first reference electrode 211 formed on the insulating layer 120, a first solid electrolyte body 212 covering the front and side surfaces of the first reference electrode 211, and a first detection electrode 213 formed on the front surface of the first solid electrolyte body 212. Similarly, as shown in FIG. 3, the second ammonia detection section 103 includes a second reference electrode 221 formed on the insulating layer 120, a second solid electrolyte body 222 covering the front and side surfaces of the second reference electrode 221, and a second detection electrode 223 formed on the front surface of the second solid electrolyte body 222.

The first reference electrode 211 and the second reference electrode 221 are formed mainly of platinum used as an electrode material and more specifically formed of a material containing Pt and zirconium oxide. The first solid electrolyte body 212 and the second solid electrolyte body 222 are formed of an oxygen ion-conductive material such as yttria-stabilized zirconia. The first detection electrode 213 and the second detection electrode 223 are formed mainly of gold used as an electrode material and more specifically formed of a material containing Au and zirconium oxide. The electrode materials of the first detection electrode 213 and the second detection electrode 223 are selected such that the first ammonia detection section 102 and the second ammonia detection section 103 differ from each other in terms of the ratio between the sensitivity to ammonia and the sensitivity to NOx.

The first ammonia detection section 102 and the second ammonia detection section 103 are covered with a porous protecting layer 230. The protecting layer 230 is configured to prevent adhesion of a poisoning material to the first detection electrode 213 and the second detection electrode 223 and to control the diffusion rate of ammonia flowing from the outside into the first ammonia detection section 102 and the second ammonia detection section 103. As described above, the first ammonia detection section 102 and the second ammonia detection section 103 function as mixed potential sensing sections.

As shown in FIG. 2, the control unit 3 includes a control circuit 180 and a microcomputer 190.

The control circuit 180 is an analog circuit disposed on a circuit board. The control circuit 180 includes an Ip1 drive circuit 181, a Vs detection circuit 182, a reference voltage comparison circuit 183, an Icp supply circuit 184, a Vp2 application circuit 185, an Ip2 detection circuit 186, a heater drive circuit 187, and an electromotive force detection circuit 188.

The pumping electrode 132, the detection electrode 142, and the pumping electrode 152 are connected to a reference potential. The pumping electrode 133 is connected to the Ip1 drive circuit 181. The reference electrode 143 is connected to the Vs detection circuit 182 and the Icp supply circuit 184. The pumping electrode 153 is connected to the Vp2 application circuit 185 and the Ip2 detection circuit 186. The heater 160 is connected to the heater drive circuit 187.

The Ip1 drive circuit 181 applies a voltage Vp1 between the pumping electrode 132 and the pumping electrode 133 to supply a first pumping current Ip1 and detects the supplied first pumping current Ip1.

The Vs detection circuit 182 detects the voltage Vs between the detection electrode 142 and the reference electrode 143 and outputs the detection result to the reference voltage comparison circuit 183.

The reference voltage comparison circuit 183 compares a reference voltage (e.g., 425 mV) with the output from the Vs detection circuit 182 (i.e., the voltage Vs) and outputs the comparison result to the Ip1 drive circuit 181. The Ip1 drive circuit 181 controls the direction and magnitude of the first pumping current Ip1 such that the voltage Vs becomes equal to the reference voltage to thereby adjust the concentration of oxygen in the first measurement chamber 121 to a prescribed value at which decomposition of NOx does not occur.

The Icp supply circuit 184 causes a weak current Icp to flow between the detection electrode 142 and the reference electrode 143. Oxygen is thereby fed from the first measurement chamber 121 to the reference oxygen chamber 146 through the solid electrolyte layer 141, and the concentration of oxygen in the reference oxygen chamber 146 is set to be a prescribed oxygen concentration serving as a reference.

The Vp2 application circuit 185 applies a constant voltage Vp2 (e.g., 450 mV) between the pumping electrode 152 and the pumping electrode 153. In the second measurement chamber 148, NOx is dissociated through the catalytic action of the pumping electrodes 152 and 153 of the second pumping cell 150. The oxygen ions obtained as a result of the dissociation migrate in the solid electrolyte layer 151 between the pumping electrode 152 and the pumping electrode 153, so that a second pumping current Ip2 flows. The Ip2 detection circuit 186 detects the second pumping current Ip2.

The heater drive circuit 187 applies a positive voltage for heater energization to one end of the heater 160, which is a heat-generating resistor, and applies a negative voltage for heater energization to the other end of the heater 160 to thereby drive the heater 160.

The electromotive force detection circuit 188 detects the electromotive force between the first reference electrode 211 and the first detection electrode 213 (hereinafter referred to as a first ammonia electromotive force) and the electromotive force between the second reference electrode 221 and the second detection electrode 223 (hereinafter referred to as a second ammonia electromotive force), and outputs signals representing the detection results to the microcomputer 190.

The microcomputer 190 includes a CPU 191, a ROM 192, a RAM 193, and a signal input/output unit 194.

The CPU 191 executes a process for controlling the sensor element unit 5 according to a program stored in the ROM 192. The signal input/output unit 194 is connected to the Ip1 drive circuit 181, the Vs detection circuit 182, the Ip2 detection circuit 186, the heater drive circuit 187, and the electromotive force detection circuit 188. The signal input/output unit 194 converts the voltage values of analog signals from the Ip1 drive circuit 181, the Vs detection circuit 182, the Ip2 detection circuit 186, and the electromotive force detection circuit 188 to digital data and outputs the digital data to the CPU 191.

The CPU 191 outputs a driving signal to the heater drive circuit 187 through the signal input/output unit 194 so as to control the electric power supplied to the heater 160 by means of pulse width modulation such that the heater 160 reaches a target temperature. To control the electric power supplied to the heater 160, any known method may be used. Specifically, the impedance of a cell (e.g., the oxygen concentration detection cell 140) included in the NOx detection section 101 is detected, and then the amount of the electric power supplied is controlled such that the impedance detected reaches a target value.

The CPU 191 reads various data from the ROM 192 and performs various computation processes on the basis of the value of the first pumping current Ip1, the value of the second pumping current Ip2, the value of the first ammonia electromotive force, and the value of the second ammonia electromotive force.

The ROM 192 stores a "first ammonia electromotive force - first ammonia concentration output relational expression," a "second ammonia electromotive force - second ammonia concentration output relational expression," a "first pumping current - oxygen concentration relational expression," a "second pumping current - NOx concentration output relational expression," a "first ammonia concentration output & second ammonia concentration output & oxygen concentration - corrected ammonia concentration relational expression," a "first ammonia concentration output & second ammonia concentration output & oxygen concentration - corrected $NO_2$ concentration relational expression," and an "NOx concentration output & corrected ammonia concentration & corrected $NO_2$ concentration - corrected NOx concentration relational expression."

The "first ammonia concentration output & second ammonia concentration output & oxygen concentration - corrected ammonia concentration relational expression" corresponds to correction expression (1) below. The "first ammonia concentration output & second ammonia concentration output & oxygen concentration - corrected $NO_2$ concentration relational expression" corresponds to correction expression (2) below. The "NOx concentration output & corrected ammonia concentration & corrected $NO_2$ concentration - corrected NOx concentration relational expression" corresponds to correction expression (3) below.

The various data may be set in the form of predetermined relational expressions as described above or may be set in other forms (for example, tables) so long as various gas concentrations can be calculated from the outputs of the sensor. Alternatively, they may be values obtained through the use of a model gas whose gas concentration is known.

The "first ammonia electromotive force - first ammonia concentration output relational expression" and the "second ammonia electromotive force - second ammonia concentration output relational expression" are expressions representing the relation between the ammonia electromotive forces outputted from the first ammonia detection section 102 and the second ammonia detection section 103 and the ammonia concentration outputs.

The "first pumping current - oxygen concentration relational expression" is an expression representing the relation between the first pumping current and the oxygen concentration (i.e., the $O_2$ concentration) in the exhaust gas. The "second pumping current - NOx concentration output relational expression" is an expression representing the relation between the second pumping current and the NOx concentration output.

The "first ammonia concentration output & second ammonia concentration output & oxygen concentration - corrected ammonia concentration relational expression" is an expression representing the relation between the first and second ammonia concentration outputs affected by the oxygen concentration, the ammonia concentration, and the $NO_2$ concentration and the corrected ammonia concentration from which the influences of the oxygen concentration and the $NO_2$ concentration have been removed. The "first ammonia concentration output & second ammonia concentration output & oxygen concentration - corrected $NO_2$ concentration relational expression" is an expression representing the relation between the first and second ammonia concentration outputs affected by the oxygen concentration, the ammonia concentration, and the $NO_2$ concentration and the corrected $NO_2$ concentration from which the influences of the oxygen concentration and the ammonia concentration have been removed. The "NOx concentration output & corrected ammonia concentration & corrected $NO_2$ concentration - corrected NOx concentration relational expression" is an expression representing the relation between the NOx concentration output affected by the ammonia concentration and the $NO_2$ concentration and the corrected NOx concentration from which the influences of the ammonia concentration and the $NO_2$ concentration have been removed.

A description will next be given of a computation process for determining the $NO_2$ concentration, the NOx concentration, and the ammonia concentration from the first pumping current Ip1, the second pumping current Ip2, the first ammonia electromotive force, and the second ammonia electromotive force. This computation process is executed by the CPU 191 of the microcomputer 190.

When the first pumping current Ip1, the second pumping current Ip2, the first ammonia electromotive force, and the second ammonia electromotive force are inputted, the CPU 191 performs a computation process for determining the oxygen concentration, the NOx concentration output, the first ammonia concentration output, and the second ammonia concentration output. Specifically, the CPU 191 calls the "first ammonia electromotive force - first ammonia concentration output relational expression," the "second ammonia electromotive force - second ammonia concentration output relational expression," the "first pumping current Ip1 - oxygen concentration relational expression," and the "second pumping current Ip2 - NOx concentration output relational expression" from the ROM 192 and then calculates the oxygen concentration and other concentration outputs using these relational expressions.

The "first ammonia electromotive force - first ammonia concentration output relational expression" and the "second ammonia electromotive force - second ammonia concentration output relational expression" are set such that, over the entire possible range of the ammonia electromotive forces outputted from the first and second ammonia detection sections 102 and 103 in their use environment, an approximately linear relation is present between each of the ammonia concentration outputs from the first and second ammonia detection sections 102 and 103 and the ammonia concentration in the measurement gas. Since these conversion expressions are used for conversion, in the correction expressions below, calculation which utilizes changes in gradient and offset is possible.

After the oxygen concentration, the NOx concentration output, the first ammonia concentration output, and the second ammonia concentration output are determined, the CPU 191 performs computations using the correction expressions described below to determine the ammonia concentration, $NO_2$ concentration, and NOx concentration in the exhaust gas.

$$\text{Correction expression (1): } x = F(A, B, D)$$

$$= (eA-c)*(jB-h-fA+d)/(eA-c-iB+g)+fA-d$$

$$\text{Correction expression (2): } y = F'(A, B, D)$$

$$= (jB-h-fA+d)/(eA-c-iB+g)$$

$$\text{Correction expression (3): } z = C-ax+by$$

In these correction expressions, x represents the ammonia concentration, y represents the $NO_2$ concentration, and z represents the NOx concentration. A represents the first ammonia concentration output, B represents the second ammonia concentration output, C represents the NOx concentration output, and D represents the oxygen concentration. F in the correction expression (1) represents that x is a function of A, B, and D, and F' in the correction expression (2) represents that y is a function of A, B, and D. a and b are correction coefficients, and c, d, e, f, g, h, i, and j are coefficients calculated using the oxygen concentration D (i.e., coefficients determined by D).

The CPU 191 determines the ammonia concentration, the $NO_2$ concentration, and the NOx concentration in the exhaust gas by substituting the first ammonia concentration output, the second ammonia concentration output, the NOx concentration output, and the oxygen concentration into the above-described correction expressions (1) to (3).

The correction expressions (1) and (2) are determined on the basis of the characteristics of the first and second ammonia detection sections 102 and 103, and the correction expression (3) is determined on the basis of the characteristics of the NOx detection section 101. The correction expressions (1) to (3) are merely examples, and other correction expressions, coefficients, etc. may be appropriately used in accordance with the characteristics of gas detection.

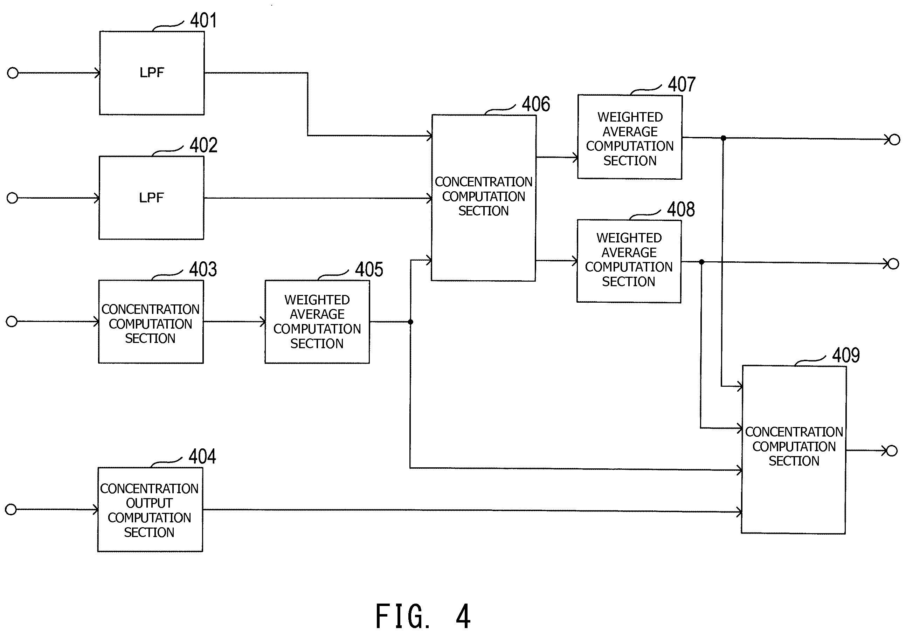

As shown in FIG. 4, the microcomputer 190 includes low-pass filters (hereinafter referred to as LPFs) 401 and 402, concentration computation sections 403, 406, and 409, a concentration output computation section 404, and weighted average computation sections 405, 407, and 408, as functional blocks realized by software processing executed by the microcomputer.

The various functions of the microcomputer are realized by a program which is stored in a non-transitory tangible recording medium and executed by the CPU. In this example, the ROM corresponds to the non-transitory tangible recording medium in which the program is stored. A method corresponding to the program is performed as a result of execution of the program. Notably, the number of microcomputer(s) constituting the control section 3 may be one or more. Also, some or all the functions executed by the microcomputer 190 may be realized by means of hardware such as one IC or a plurality of ICs.

The LPF 401 acquires digital data representing the first ammonia electromotive force and performs digital processing of extracting a low-frequency component whose frequency is equal to or lower than a predetermined cutoff frequency from a waveform which shows a change in the first ammonia electromotive force with time.

The LPF 402 acquires digital data representing the second ammonia electromotive force and performs digital processing of extracting a low-frequency component whose frequency is equal to or lower than a predetermined cutoff frequency from a waveform which shows a change in the second ammonia electromotive force with time.

The concentration computation section 403 acquires digital data representing the first pumping current Ip1 and computes the oxygen concentration on the basis of the "first pumping current - oxygen concentration relational expression."

The concentration output computation section 404 acquires digital data representing the second pumping current Ip2 and computes the NOx concentration output on the basis of the "second pumping current Ip2 - NOx concentration output relational expression."

The weighted average computation section 405 acquires digital data representing the oxygen concentration from the concentration computation section 403 and computes the weighted average of the oxygen concentration represented by the digital data acquired this time (hereinafter referred to as "latest oxygen concentration") and the oxygen concentration computed last time by the weighted average computation section 405 (hereinafter referred to as "previous oxygen concentration"). Specifically, the weighted average computation section 405 computes the weighted average in accordance with an expression of $(xA+yB)/(x+y)$ where A represents the latest oxygen concentration, B represents the previous oxygen concentration, x represents a weighting factor for the latest oxygen concentration, and y represents a weighting factor for the previous oxygen concentration. In the present embodiment, for example, the weighting factor for the latest oxygen concentration is 1, and the weighting factor for the previous oxygen concentration is 40. As a result, the speed of response of the first pumping current Ip1 to the oxygen concentration at the NOx detection section 101 can be rendered equal to the speed of response of the first and second ammonia electromotive forces to the $NO_2$ concentration and the ammonia concentration at the first and second ammonia detection sections 102 and 103.

The concentration computation section 406 acquires digital data representing the first ammonia electromotive force from the LPF 401, acquires digital data representing the second ammonia electromotive force from the LPF 402, and acquires digital data representing the oxygen concentration from the weighted average computation section 405. The concentration computation section 406 computes the ammonia concentration on the basis of the "first ammonia electromotive force - first ammonia concentration output relational expression," the "second ammonia electromotive force - second ammonia concentration output relational expression," and the "first ammonia concentration output & second ammonia concentration output & oxygen concentration - corrected ammonia concentration relational expression." Also, the concentration computation section 406 computes the $NO_2$ concentration on the basis of the "first ammonia concentration output & second ammonia concentration output & oxygen concentration - corrected $NO_2$ concentration relational expression."

The weighted average computation section 407 acquires digital data representing the ammonia concentration from the concentration computation section 406 and computes the weighted average of the ammonia concentration represented by the digital data acquired this time (hereinafter referred to as "latest ammonia concentration") and the ammonia concentration computed last time by the weighted average computation section 407 (hereinafter referred to as "previous ammonia concentration"). In the present embodiment, for example, the weighting factor for the latest ammonia concentration is 1, and the weighting factor for the previous ammonia concentration is 20. As a result, the speed of response of the second pumping current Ip2 to the NOx concentration at the NOx detection section 101 can be rendered equal to the speed of response of the first and second ammonia electromotive forces to the ammonia concentration at the first and second ammonia detection sections 102 and 103.

The weighted average computation section 408 acquires digital data representing the $NO_2$ concentration from the concentration computation section 406 and computes the weighted average of the $NO_2$ concentration represented by the digital data acquired this time (hereinafter referred to as "latest $NO_2$ concentration") and the $NO_2$ concentration computed last time by the weighted average computation section 408 (hereinafter referred to as "previous $NO_2$ concentration"). In the present embodiment, for example, the weighting factor for the latest $NO_2$ concentration is 1, and the weighting factor for the previous $NO_2$ concentration is 20. As a result, the speed of response of the second pumping current Ip2 to the NOx concentration at the NOx detection section 101 can be rendered equal to the speed of response of the first and second ammonia electromotive forces to the $NO_2$ concentration at the first and second ammonia detection sections 102 and 103.

The concentration computation section 409 acquires digital data representing the ammonia concentration from the weighted average computation section 407, acquires digital data representing the $NO_2$ concentration from the weighted average computation section 408, acquires digital data representing the oxygen concentration from the weighted average computation section 405, and acquires digital data representing the NOx concentration output form the concentration output computation section 404. The concentration computation section 409 computes the NOx concentration on the basis of the "NOx concentration output & corrected ammonia concentration & corrected $NO_2$ concentration - corrected NOx concentration relational expression."

The multi-gas detection apparatus 100 configured as described above computes the concentrations of ammonia, $NO_2$, and NOx contained in exhaust gas through use of the multi-gas sensor 2 including the NOx detection section 101, the first ammonia detection section 102, and the second ammonia detection section 103.

The NOx detection section 101 has the first pumping cell 130 and the second pumping cell 150. The first pumping current Ip1 flows through the first pumping cell 130. Specifically, the first pumping cell 130 pumps out oxygen contained in the exhaust gas introduced into the first measurement chamber 121 or pumps oxygen into the first measurement chamber 121, whereby the first pumping current Ip1 whose value changes in accordance with the oxygen concentration in the exhaust gas flows through the first pumping cell 130. The second pumping current Ip2 flows through the second pumping cell 150. The value of the second pumping current Ip2 changes in accordance with the concentration of NOx contained in the exhaust gas whose oxygen concentration has been adjusted in the first measurement chamber 121 by the first pumping cell 130.

The first ammonia detection section 102 and the second ammonia detection section 103 output the first ammonia electromotive force and the second ammonia electromotive force, respectively, whose values change in accordance with the concentrations of ammonia and $NO_2$ contained in the exhaust gas.

The multi-gas detection apparatus 100 includes the concentration output computation section 404 and the concentration computation sections 403, 406, and 409. The concentration computation sections 403 and 406 are configured to compute the ammonia concentration and the $NO_2$ concentration in the exhaust gas on the basis of the value of the first pumping current Ip1 and the values of the first ammonia electromotive force and the second ammonia electromotive force.

The concentration output computation section 404 and the concentration computation section 409 are configured to compute the NOx concentration in the exhaust gas on the basis of the ammonia concentration and the $NO_2$ concentration computed by the concentration computation sections 403 and 406 and the value of the second pumping current Ip2.

The multi-gas detection apparatus 100 includes the weighted average computation sections 405, 407, and 408.

The weighted average computation section 405 is configured to delay, by means of digital filter processing, the timing at which a change in the value of the first pumping current Ip1 flowing through the first pumping cell 130 is reflected on the computation of the $NO_2$ concentration and the ammonia concentration by the concentration computation section 406. As a result, by means of digital filter processing, the weighted average computation section 405 decreases the response speed of the first pumping current Ip1 which changes in accordance with the oxygen concentration in the exhaust gas.

The weighted average computation sections 407 and 408 are configured to delay, by means of digital filter processing, the timings at which changes in the values of the first and second ammonia electromotive forces generated at the first and second ammonia detection sections 102 and 103 are reflected on the computation of the NOx concentration by the concentration computation section 409. As a result, by means of digital filter processing, the weighted average computation sections 407 and 408 decrease the response speeds of the first and second ammonia electromotive forces which change in accordance with the ammonia concentration and the $NO_2$ concentration in the exhaust gas.

Notably, the multi-gas sensor 2 (the sensor element unit 5) of the present embodiment is configured such that the NOx detection section 101 configured to adjust the oxygen concentration in the measurement gas and decompose NOx in the gas whose oxygen concentration has been adjusted is unified with the first and second mixed-potential-type ammonia detection sections 102 and 103, and all the detection sections 101, 102, and 103 are disposed at approximately the same position in relation to the space through which the measurement gas (exhaust gas) flows. In such a configuration, there is a tendency that, of the response speeds of the first pumping current Ip1, the second pumping current Ip2, and the first and second ammonia electromotive forces, the response speed of the first pumping current Ip1 is the highest, the response speed of the first and second ammonia electromotive forces is the second highest, and the response speed of the second pumping current Ip2 is the lowest.

As described above, the multi-gas detection apparatus 100 decreases the response speed of the first pumping current Ip1 by using the weighted average computation section 405. Therefore, the difference between the response speed of the first pumping current Ip1 and the response speed of the first and second ammonia electromotive forces can be reduced. As a result, when computing the ammonia concentration and the $NO_2$ concentration by using the value of the first pumping current Ip1 and the values of the first and second ammonia electromotive forces, the multi-gas detection apparatus 100 can suppress the influence of the difference between the response speed of the first pumping current Ip1 and the response speed of the first and second ammonia electromotive forces, to thereby improve the computation accuracy of the ammonia concentration and the $NO_2$ concentration.

Further, the multi-gas detection apparatus 100 decreases the response speeds of the first and second ammonia electromotive forces by using the weighted average computation sections 407 and 408. Therefore, the difference between the response speed of the first and second ammonia electromotive forces and the response speed of the second pumping current Ip2 can be reduced. As a result, when computing the NOx concentration by using the ammonia concentration and the NO$_2$ concentration, the multi-gas detection apparatus 100 can suppress the influence of the difference between the response speed of the first and second ammonia electromotive forces and the response speed of the second pumping current Ip2, to thereby improve the computation accuracy of the NOx concentration.

Also, in the multi-gas detection apparatus 100, the digital filter processing performed by the weighted average computation section 405 and the digital filter processing performed by the weighted average computation sections 407 and 408 are weighted averaging. Since the multi-gas detection apparatus can decrease the response speeds by weighted averaging which is simple digital filter processing, the processing load of the multi-gas detection apparatus 100 can be reduced.

Notably, the microcomputer 190 corresponds to the concentration computation apparatus in the claims; the first measurement chamber 121 corresponds to the measurement chamber in the claims; the exhaust gas corresponds to the measurement gas in the claims; and the NOx detection section 101 corresponds to the first detection section in the claims.

Also, the first ammonia electromotive force and the second ammonia electromotive force correspond to the concentration signal in the claims; and the first ammonia detection section 102 and the second ammonia detection section 103 correspond to the second detection section in the claims.

Also, the weighted average computation section 405 corresponds to the first delaying section in the claims; the weighted average computation sections 407 and 408 correspond to the second delaying section in the claims; the concentration computation sections 403 and 406 correspond to the first concentration computation section in the claims; and the concentration output computation section 404 and the concentration computation section 409 correspond to the second concentration computation section in the claims.

(Second Embodiment)

A second embodiment of the present invention will now be described with reference to the drawings. Portions of the second embodiment different from those of the first embodiment will be described. The components which are common between the embodiments are dented by the same reference numerals.

The multi-gas detection apparatus of the second embodiment differs from the multi-gas detection apparatus of the first embodiment in the point that the software processing executed by the microcomputer 190 differs from that executed in the first embodiment.

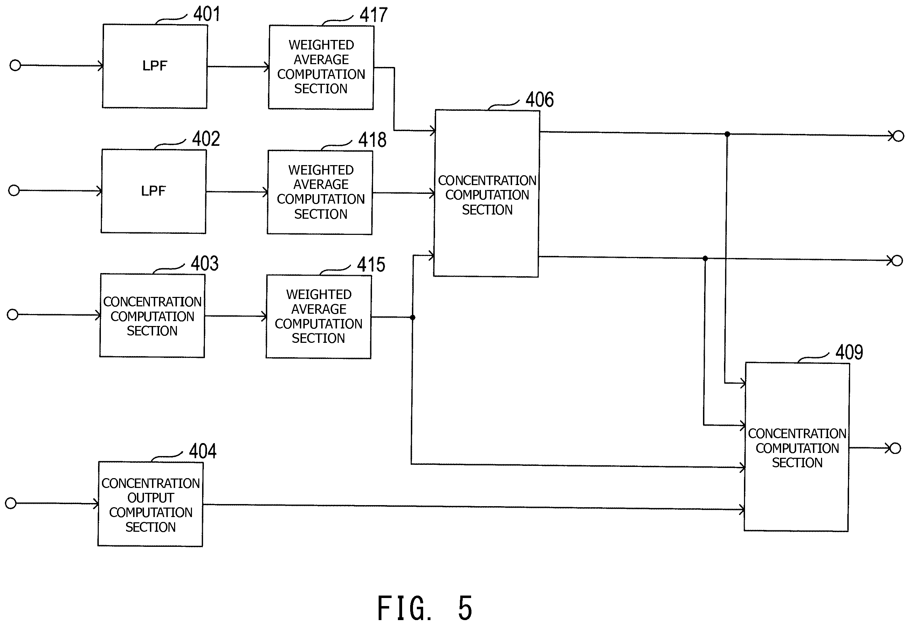

Specifically, the microcomputer 190 of the second embodiment differs from the microcomputer 190 of the first embodiment in the point that weighted average computation sections 415, 417, and 418 are provided instead of the weighted average computation sections 405, 407, and 408 as shown in FIG. 5.

The weighted average computation section 415 acquires digital data representing the oxygen concentration from the concentration computation section 403 and computes the weighted average of the latest oxygen concentration and the previous oxygen concentration. In the present embodiment, for example, the weighting factor for the latest oxygen concentration is 1, and the weighting factor for the previous oxygen concentration is 60. As a result, the speed of response of the first pumping current Ip1 to the oxygen concentration at the NOx detection section 101 can be rendered equal to the speed of response of the second pumping current Ip2 to the NOx concentration at the NOx detection section 101.

The weighted average computation section 417 acquires digital data representing the first ammonia electromotive force from the LPF 401 and computes the weighted average of the first ammonia electromotive force represented by the digital data acquired this time (hereinafter referred to as the "latest first ammonia electromotive force") and the first ammonia electromotive force computed by the weighted average computation section 417 last time ((hereinafter referred to as the "previous first ammonia electromotive force"). In the present embodiment, for example, the weighting factor for the latest first ammonia electromotive force is 1, and the weighting factor for the previous first ammonia electromotive force is 20. As a result, the speed of response of the second pumping current Ip2 to the NOx concentration at the NOx detection section 101 can be rendered equal to the speed of response of the first ammonia electromotive force to the NO$_2$ concentration and the ammonia concentration at the first ammonia detection section 102.

The weighted average computation section 418 acquires digital data representing the second ammonia electromotive force from the LPF 402 and computes the weighted average of the second ammonia electromotive force represented by the digital data acquired this time (hereinafter referred to as the "latest second ammonia electromotive force") and the second ammonia electromotive force computed by the weighted average computation section 418 last time ((hereinafter referred to as the "previous second ammonia electromotive force"). In the present embodiment, for example, the weighting factor for the latest second ammonia electromotive force is 1, and the weighting factor for the previous second ammonia electromotive force is 20. As a result, the speed of response of the second pumping current Ip2 to the NOx concentration at the NOx detection section 101 can be rendered equal to the speed of response of the second ammonia electromotive force to the $NO_2$ concentration and the ammonia concentration at the second ammonia detection section 103.

The concentration computation section 406 acquires digital data representing the first ammonia electromotive force from the weighted average computation section 417, acquires digital data representing the second ammonia electromotive force from the weighted average computation section 418, and acquires digital data representing the oxygen concentration from the weighted average computation section 415. The concentration computation section 406 computes the ammonia concentration on the basis of the "first ammonia electromotive force - first ammonia concentration output relational expression," the "second ammonia electromotive force - second ammonia concentration output relational expression," and the "first ammonia concentration output & second ammonia concentration output & oxygen concentration output - corrected ammonia concentration relational expression." Also, the concentration computation section 406 computes the $NO_2$ concentration on the basis of the "first ammonia concentration output & second ammonia concentration output & oxygen concentration - corrected $NO_2$ concentration relational expression."

The concentration computation section 409 acquires digital data representing the ammonia concentration from the concentration computation section 406, acquires digital data representing the $NO_2$ concentration from the concentration computation section 406, acquires digital data representing the oxygen concentration from the weighted average computation section 415, and acquires digital data representing the NOx concentration output form the concentration output computation section 404. The concentration computation section 409 computes the NOx concentration on the basis of the "NOx concentration output & corrected ammonia concentration & corrected $NO_2$ concentration - corrected NOx concentration relational expression."

The multi-gas detection apparatus configured as described includes the weighted average computation sections 415, 417, and 418.

The weighted average computation section 415 is configured to delay, by means of digital filter processing, the timing at which a change in the value of the first pumping current Ip1 flowing through the first pumping cell 130 is reflected on the computation of the $NO_2$ concentration and the ammonia concentration by the concentration computation section 406. As a result, by means of digital filter processing, the weighted average computation section 415 decreases the response speed of the first pumping current Ip1 which changes in accordance with the oxygen concentration in the exhaust gas.

The weighted average computation sections 417 and 418 are configured to delay, by means of digital filter processing, the timings at which changes in the values of the first and second ammonia electromotive forces generated at the first and second ammonia detection sections 102 and 103 are reflected on the computation of the NOx concentration by the concentration computation section 409. As a result, by means of digital filter processing, the weighted average computation sections 417 and 418 decrease the response speeds of the first and second ammonia electromotive forces which change in accordance with the ammonia concentration and the $NO_2$ concentration in the exhaust gas.

Thus, the multi-gas detection apparatus can suppress the influence of the difference between the response speed of the first pumping current Ip1 and the response speed of the first and second ammonia electromotive forces, to thereby improve the computation accuracy of the ammonia concentration and the $NO_2$ concentration. Further, the multi-gas detection apparatus can suppress the influence of the difference between the response speed of the first and second ammonia electromotive forces and the response speed of the second pumping current Ip2, to thereby improve the computation accuracy of the NOx concentration.

Notably, the weighted average computation section 415 correspond to the first delaying section in the claims; and the weighted average computation sections 417 and 418 correspond to the second delaying section in the claims.

While the embodiments of the present invention have been described, the present invention is not limited to these embodiments. The present invention can be implemented in modified forms.

For example, in the above-described embodiments, the digital filter processing is weighted averaging. However, the digital filter processing is not limited to the weighted averaging. Any of other types of digital filter processing may be used so long as the employed digital filter processing can decrease the response speeds. An example of such digital filter processing is an IIR filter. IIR is an abbreviation for infinite impulse response.

In the above-described embodiments, the weighted average computation section 405 performs the digital filter processing on the digital data computed by the concentration computation section 403. However, the embodiments may be modified such that the processing for weighted averaging is performed on the digital data representing the first pumping current Ip1, and the concentration computation section 403 computes the oxygen concentration by using the digital data after having undergone the processing for weighted averaging.

In the above-described second embodiment, the weighted average computation sections 417 and 418 perform the digital filter processing on the digital data output from the LPFs 401 and 402. However, the second embodiment may be modified such that the processing for weighted averaging is performed on the digital data representing the first and second ammonia electromotive forces, and the digital data after having undergone the processing for weighted averaging are output to the LPFs 401 and 402.

The function of a single component in the above-described embodiments may be distributed to a plurality of components, or functions of a plurality of components may be integrated into one component. Part of the configuration in each of the above-described embodiments may be omitted. At least part of the configuration in each of the above-described embodiments may be added to the configuration of another embodiment or may replace the configuration of another embodiment. Any embodiments included in the technical ideas specified by the wording of the claims are defined as embodiments of the present disclosure.

The present disclosure can be implemented as various forms such as the microcomputer 190 described above and as a system including the microcomputer 190 as a component, a program that causes the microcomputer 190 to function as a computer, a non-transitory tangible recording medium, e.g., a semiconductor memory, in which the program is stored, and a concentration computation method.

[Description of Reference Numerals]

2 ⋯ multi-gas sensor, 3 ⋯ control section, 5 ⋯ sensor element unit, 101 ⋯ NOx detection section, 102 ⋯ first ammonia detection section, 103 ⋯ second ammonia detection section, 121 ⋯ first measurement chamber, 130 ⋯ first pumping cell, 150 ⋯ second pumping cell, 190 ⋯ microcomputer, 405, 407, 408, 415, 417, 418 ⋯ weighted average computation section.

**Claims**

1. A concentration computation apparatus (3) for computing the concentrations of ammonia, nitrogen dioxide, and nitrogen oxide contained in a target gas, said concentration computation apparatus (3) receiving signals from a multi-gas sensor (2) that includes: a first detection section (101) having a first pumping cell (130) which pumps out oxygen contained in the target gas introduced into a measurement chamber (121) or pumps oxygen into the measurement chamber (121) and through which a first pumping current (Ip1) flows as a result of the pumping of oxygen, the value of the first pumping current (Ip1) changing in accordance with the concentration of oxygen contained in the target gas, and a second pumping cell (150) through which a second pumping current (Ip2) flows, the value of the second pumping current (Ip2) changing in accordance with the concentration of nitrogen oxide contained in the target gas whose oxygen concentration has been adjusted in the measurement chamber (121) by the first pumping cell (130); and a second detection section (102, 103) which outputs two concentration signals whose values change in accordance with the concentrations of ammonia and nitrogen dioxide contained in the target gas, the concentration computation apparatus **characterised in that** it comprises:

   a first delaying section (405) which is configured to delay by digital filter processing the timing at which a change in the value of the first pumping current (Ip1) is reflected on the computation of the ammonia and nitrogen dioxide concentrations and
   a second delaying section (407, 408) which is configured to delay, by digital filter processing the timings at which changes in the values of the concentration signals are reflected on the computation of the nitrogen oxide concentration.

2. The concentration computation apparatus (3) according to claim 1, further comprising:

   a first concentration computation section (406) which computes an ammonia concentration and a nitrogen dioxide concentration in the target gas on a basis of a value of the first pumping current (Ip1) and a value of the concentration signals, and
   a second concentration computation section (409) which computes the nitrogen oxide concentration in the target gas on a basis of the ammonia concentration and the nitrogen dioxide concentration computed by the first concentration computation section (406) and the second pumping current (Ip2),
   the first delaying section (405) delays, by digital filter processing, a timing at which a change in the value of the first pumping current (Ip1) at the first detection section (101) is reflected on the computation of the nitrogen dioxide concentration and the ammonia concentration performed by the first concentration computation section (406), and
   the second delaying section (407, 408) delays, by digital filter processing, a timing at which a change in the value of the concentration signals at the second detection section (102, 103) is reflected on the computation of the nitrogen oxide concentration performed by the second concentration computation section (409).

3. The concentration computation apparatus (3) according to claim 1, wherein at least one of the digital filter processing performed by the first delaying section (405) and the digital filter processing performed by the second delaying section (407, 408) is weighted averaging.

4. The concentration computation apparatus (3) according to claim 2, wherein at least one of the digital filter processing performed by the first delaying section (405) and the digital filter processing performed by the second delaying section (407, 408) is weighted averaging.

5. A multi-gas detection apparatus (100) comprising:

a concentration computation apparatus (3) that computes concentrations of ammonia, nitrogen dioxide, and nitrogen oxide contained in a target gas; and
a multi-gas sensor (2) that sends signals to the concentration computation apparatus (3), wherein
the concentration computation apparatus (3) computes the concentrations by using signals sent from the multi-gas sensor (2),
the multi-gas sensor includes (2);

a first detection section (101) having a first pumping cell (130) which pumps out oxygen contained in the target gas introduced into a measurement chamber (121) or pumps oxygen into the measurement chamber (121) and through which a first pumping current (Ip1) flows as a result of the pumping of oxygen, the value of the first pumping current (Ip1) changing in accordance with the concentration of oxygen contained in the target gas, and a second pumping cell (150) through which a second pumping current (Ip2) flows, the value of the second pumping current (Ip2) changing in accordance with the concentration of nitrogen oxide contained in the target gas whose oxygen concentration has been adjusted in the measurement chamber (121) by the first pumping cell (130), and
a second detection section (102, 103) which outputs two concentration signals whose values change in accordance with the concentrations of ammonia and nitrogen dioxide contained in the target gas;

the multi-gas detection apparatus (100) being **characterised in that** the concentration computation apparatus (3) includes;

a first delaying section (405) which is configured to delay, by digital filter processing, the timing at which a change in the value of the first pumping current (Ip1) is reflected on the computation of the ammonia and nitrogen dioxide concentrations and
a second delaying section (407, 408) which is configured to delay, by digital filter processing the timings at which changes in the values of the concentration signals are reflected on the computation of the nitrogen oxide concentration.

## Patentansprüche

1. Konzentrationsberechnungsvorrichtung (3) zum Berechnen der Konzentrationen von Ammoniak, Stickstoffdioxid und Stickstoffoxid, die in einem Zielgas enthalten sind, wobei die Konzentrationsberechnungsvorrichtung (3) Signale von einem Multigassensor (2) erhält, der Folgendes enthält: einen ersten Erfassungsabschnitt (101) mit einer ersten Pumpzelle (130), die Sauerstoff auspumpt, der in dem Zielgas enthalten ist, das in eine Messkammer (121) eingeführt wird, oder Sauerstoff in die Messkammer (121) pumpt und durch die ein erster Pumpstrom (Ip1) fließt als Resultat vom Pumpen des Sauerstoffs, wobei sich der Wert des ersten Pumpstroms (Ip1) in Übereinstimmung mit der Sauerstoffkonzentration verändert, die in dem Zielgas enthalten ist, und einer zweiten Pumpzelle (150), durch die ein zweiter Pumpstrom (Ip2) fließt, wobei sich der Wert des zweiten Pumpstroms (Ip2) in Übereinstimmung mit der Stickstoffoxidkonzentration verändert, die in dem Zielgas enthalten ist, dessen Sauerstoffkonzentration in der Messkammer (121) von der ersten Pumpzelle (130) angepasst wurde; und einen zweiten Erfassungsabschnitt (102, 103), der zwei Konzentrationssignale ausgibt, dessen Werte sich in Übereinstimmung mit der Ammoniak- und Stickstoffdioxidkonzentration verändern, die in dem Zielgas enthalten sind, wobei die Konzentrationsberechnungsvorrichtung **dadurch gekennzeichnet ist, dass** sie Folgendes umfasst:

einen ersten Verzögerungsabschnitt (405), der konfiguriert ist, um durch Digitalfilterverarbeitung den Zeitpunkt zu verzögern, an dem eine Wertveränderung des ersten Pumpstroms (Ip1) auf der Berechnung der Ammoniak- und Stickstoffdioxidkonzentrationen widerspiegelt wird, und
einen zweiten Verzögerungsabschnitt (407, 408), der konfiguriert ist, um durch Digitalfilterverarbeitung die Zeitpunkte zu verzögern, an denen Wertveränderungen der Konzentrationssignale auf der Berechnung der Stickstoffoxidkonzentration widerspiegelt werden.

2. Konzentrationsberechnungsvorrichtung (3) nach Anspruch 1, ferner umfassend:

einen ersten Konzentrationsberechnungsabschnitt (406), der eine Ammoniakkonzentration und eine Stickstoffdioxidkonzentration in dem Zielgas auf Grundlage eines Werts des ersten Pumpstroms (Ip1) und eines Werts des Konzentrationssignals berechnet, und

einen zweiten Konzentrationsberechnungsabschnitt (409), der die Stickstoffoxidkonzentration in dem Zielgas auf Grundlage der Ammoniakkonzentration und der Stickstoffdioxidkonzentration berechnet, die von dem ersten Konzentrationsberechnungsabschnitt (406) und dem zweiten Pumpstrom (Ip2) berechnet werden,

der erste Verzögerungsabschnitt (405) verzögert durch Digitalfilterverarbeitung einen Zeitpunkt, an dem eine Wertveränderung des ersten Pumpstroms (Ip1) an dem ersten Erfassungsabschnitt (101) auf der Berechnung der Stickstoffdioxidkonzentration und der Ammoniakkonzentration widerspiegelt wird, die von dem ersten Konzentrationsberechnungsabschnitt (406) ausgeführt wird, und

der zweite Verzögerungsabschnitt (407, 408) verzögert durch Digitalfilterverarbeitung einen Zeitpunkt, an dem eine Wertveränderung des Konzentrationssignals an dem zweiten Erfassungsabschnitt (102, 103) auf der Berechnung der Stickstoffoxidkonzentration widerspiegelt wird, die von dem zweiten Konzentrationsberechnungsabschnitt (409) ausgeführt wird.

3. Konzentrationsberechnungsvorrichtung (3) nach Anspruch 1, wobei mindestens eine der Digitalfilterverarbeitung, die von dem ersten Verzögerungsabschnitt (405) ausgeführt wird, und der Digitalfilterverarbeitung, die von dem zweiten Verzögerungsabschnitt (407, 408) ausgeführt wird, gewichtete Mittelwertbildung ist.

4. Konzentrationsberechnungsvorrichtung (3) nach Anspruch 2, wobei mindestens eine der Digitalfilterverarbeitung, die von dem ersten Verzögerungsabschnitt (405) ausgeführt wird, und der Digitalfilterverarbeitung, die von dem zweiten Verzögerungsabschnitt (407, 408) ausgeführt wird, gewichtete Mittelwertbildung ist.

5. Multigaserfassungsvorrichtung (100), umfassend:

eine Konzentrationsberechnungsvorrichtung (3), die Konzentrationen von Ammoniak, Stickstoffdioxid und Stickstoffoxid, die in einem Zielgas enthalten sind, berechnet; und

einen Multigassensor (2), der Signale an die Konzentrationsberechnungsvorrichtung (3) sendet, wobei die Konzentrationsberechnungsvorrichtung (3) die Konzentrationen unter Verwendung von Signalen berechnet, die von dem Multigassensor (2) gesendet werden,

der Multigassensor (2) Folgendes enthält;

einen ersten Erfassungsabschnitt (101) mit einer ersten Pumpzelle (130), die Sauerstoff auspumpt, der in dem Zielgas enthalten ist, das in eine Messkammer (121) eingeführt wird, oder Sauerstoff in die Messkammer (121) pumpt und durch die ein erster Pumpstrom (Ip1) fließt als Resultat vom Pumpen des Sauerstoffs, wobei sich der Wert des ersten Pumpstroms (Ip1) in Übereinstimmung mit der Sauerstoffkonzentration verändert, die in dem Zielgas enthalten ist, und einer zweiten Pumpzelle (150), durch die ein zweiter Pumpstrom (Ip2) fließt, wobei sich der Wert des zweiten Pumpstroms (Ip2) in Übereinstimmung mit der Stickstoffoxidkonzentration verändert, die in dem Zielgas enthalten ist, dessen Sauerstoffkonzentration in der Messkammer (121) von der ersten Pumpzelle (130) angepasst wurde, und

einen zweiten Erfassungsabschnitt (102, 103), der zwei Konzentrationssignale ausgibt, dessen Werte sich in Übereinstimmung mit der Ammoniak- und Stickstoffdioxidkonzentration verändern, die in dem Zielgas enthalten sind;

wobei die Multigaserfassungsvorrichtung (100) **dadurch gekennzeichnet ist, dass** die Konzentrationsberechnungsvorrichtung (3) Folgendes enthält;

einen ersten Verzögerungsabschnitt (405), der konfiguriert ist, um durch Digitalfilterverarbeitung den Zeitpunkt zu verzögern, an dem eine Wertveränderung des ersten Pumpstroms (Ip1) auf der Berechnung der Ammoniak- und Stickstoffdioxidkonzentrationen widerspiegelt wird, und

einen zweiten Verzögerungsabschnitt (407, 408), der konfiguriert ist, um durch Digitalfilterverarbeitung die Zeitpunkte zu verzögern, an denen Wertveränderungen der Konzentrationssignale auf der Berechnung der Stickstoffoxidkonzentration widerspiegelt werden.

**Revendications**

**1.** Appareil de calcul de concentration (3) pour calculer les concentrations en ammoniac, dioxyde d'azote, et oxyde d'azote contenues dans un gaz cible, ledit appareil de calcul de concentration (3) recevant des signaux à partir d'un capteur de gaz multiples (2) qui inclut: une première section de détection (101) ayant une première cellule de pompage (130) qui aspire de l'oxygène contenu dans le gaz cible introduit dans une chambre de mesure (121) ou pompe de l'oxygène dans la chambre de mesure (121) et à travers laquelle un premier courant de pompage (Ip1) s'écoule en conséquence du pompage de l'oxygène, la valeur du premier courant de pompage (Ip1) changeant conformément à la concentration en oxygène contenue dans le gaz cible, et une seconde cellule de pompage (150) à travers laquelle un second courant de pompage (Ip2) s'écoule, la valeur du second courant de pompage (Ip2) changeant conformément à la concentration en oxyde d'azote contenue dans le gaz cible dont la concentration en oxygène a été ajustée dans la chambre de mesure (121) par la première cellule de pompage (130) ; et une seconde section de détection (102, 103) qui produit en sortie deux signaux de concentration dont les valeurs changent conformément aux concentrations en ammoniac et dioxyde d'azote contenues dans le gaz cible, l'appareil de calcul de concentration étant **caractérisé en ce qu'**il comprend :

une première section retardatrice (405) qui est configurée pour retarder, par traitement de filtrage numérique, l'instant auquel un changement de la valeur du premier courant de pompage (Ip1) est reflété sur le calcul des concentrations en ammoniac et dioxyde d'azote, et
une seconde section retardatrice (407, 408) qui est configurée pour retarder, par traitement de filtrage numérique, les instants auxquels des changements des valeurs des signaux de concentration sont reflétés sur le calcul de la concentration en oxyde d'azote.

**2.** Appareil de calcul de concentration (3) selon la revendication 1, comprenant en outre :

une première section de calcul de concentration (406) qui calcule une concentration en ammoniac et une concentration en dioxyde d'azote dans le gaz cible sur la base d'une valeur du premier courant de pompage (Ip1) et d'une valeur des signaux de concentration, et
une seconde section de calcul de concentration (409) qui calcule la concentration en oxyde d'azote dans le gaz cible sur la base de la concentration en ammoniac et de la concentration en dioxyde d'azote calculées par la première section de calcul de concentration (406) et du second courant de pompage (Ip2),
la première section retardatrice (405) retarde, par traitement de filtrage numérique, un instant auquel un changement de la valeur du premier courant de pompage (Ip1) à la première section de détection (101) est reflété sur le calcul de la concentration en dioxyde d'azote et de la concentration en ammoniac réalisé par la première section de calcul de concentration (406), et
la seconde section retardatrice (407, 408) retarde, par traitement de filtrage numérique, un instant auquel un changement de la valeur des signaux de concentration à la seconde section de détection (102, 103) est reflété sur le calcul de la concentration en oxyde d'azote réalisé par la seconde section de calcul de concentration (409).

**3.** Appareil de calcul de concentration (3) selon la revendication 1, dans lequel au moins un du traitement de filtrage numérique réalisé par la première section retardatrice (405) et du traitement de filtrage numérique réalisé par la seconde section retardatrice (407, 408) fait l'objet d'un calcul de moyenne pondérée.

**4.** Appareil de calcul de concentration (3) selon la revendication 2, dans lequel au moins un du traitement de filtrage numérique réalisé par la première section retardatrice (405) et du traitement de filtrage numérique réalisé par la seconde section retardatrice (407, 408) fait l'objet d'un calcul de moyenne pondérée.

**5.** Appareil de détection de gaz multiples (100), comprenant :

un appareil de calcul de concentration (3) qui calcule des concentrations en ammoniac, dioxyde d'azote, et oxyde d'azote contenues dans un gaz cible ; et
un capteur de gaz multiples (2) qui envoie des signaux à l'appareil de calcul de concentration (3), dans lequel l'appareil de calcul de concentration (3) calcule les concentrations en utilisant des signaux envoyés à partir du capteur de gaz multiples (2),
le capteur de gaz multiples inclut (2) ;

une première section de détection (101) ayant une première cellule de pompage (130) qui aspire de l'oxygène contenu dans le gaz cible introduit dans une chambre de mesure (121) ou pompe de l'oxygène dans la chambre de mesure (121) et à travers laquelle un premier courant de pompage (Ip1) s'écoule en conséquence du pompage de l'oxygène, la valeur du premier courant de pompage (Ip1) changeant conformément à la concentration en oxygène contenue dans le gaz cible, et une seconde cellule de pompage (150) à travers laquelle un second courant de pompage (Ip2) s'écoule, la valeur du second courant de pompage (Ip2) changeant conformément à la concentration en oxyde d'azote contenue dans le gaz cible dont la concentration en oxygène a été ajustée dans la chambre de mesure (121) par la première cellule de pompage (130), et

une seconde section de détection (102, 103) qui produit en sortie deux signaux de concentration dont les valeurs changent conformément aux concentrations en ammoniac et dioxyde d'azote contenues dans le gaz cible ;

l'appareil de détection de gaz multiples (100) étant **caractérisé en ce que** l'appareil de calcul de concentration (3) inclut :

une première section retardatrice (405) qui est configurée pour retarder, par traitement de filtrage numérique, l'instant auquel un changement de la valeur du premier courant de pompage (Ip1) est reflété sur le calcul des concentrations en ammoniac et dioxyde d'azote, et

une seconde section retardatrice (407, 408) qui est configurée pour retarder, par traitement de filtrage numérique, les instants auxquels des changements des valeurs des signaux de concentration sont reflétés sur le calcul de la concentration en oxyde d'azote.

O

41        41        41        2

33                            32

36                            37

35                            38

34
38                            31

5B        5A

18
17
16
10                            5
15
11                            14
19
13
12                            21

22

O

# FIG. 1

FIG. 2

FIG. 3

FIG. 4

EP 3 293 516 B1

FIG. 5

**EP 3 293 516 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2015034814 A